# EUROPEAN PATENT SPECIFICATION

(11) **EP 3 375 427 B1**
(45) Date of publication and mention of the grant of the patent: **21.06.2023**
(21) Application number: 16863698.3
(22) Date of filing: 13.11.2016
(51) Int. Cl.: A61J 1/14, A61J 1/20, A61J 1/06, A61J 1/10, A61M 5/14

(54) **DRUG MIXER, HARD DUAL-PORT MEMBER, AND SOFT INFUSION BAG**
ARZNEIMITTELMISCHER, HARTES DOPPELANSCHLUSSELEMENT UND WEICHER INFUSIONSBEUTEL
MÉLANGEUR DE MÉDICAMENT, ÉLÉMENT DUR À DEUX ORIFICES, ET POCHE D'INFUSION SOUPLE

(30) Priority: 13.11.2015 CN 201510781507; 13.11.2015 CN 201510781506; 13.11.2015 CN 201510780465
(43) Date of publication of application: 19.09.2018
(73) Proprietor: Chongqing Lummy Pharmaceutical Co., Ltd., Chongqing 401123 (CN)
(72) Inventor: ZHANG, Yun, Chongqing 401123 (CN); LI, Ke, Chongqing 401123 (CN)
(74) Representative: Huang, Liwei
(86) International application number: PCT/CN2016/105564
(87) International publication number: WO 2017/080522

(56) References cited:
- EP-A1- 2 881 101
- CN-A- 102 499 888
- CN-A- 105 232 329
- CN-A- 105 232 330
- CN-A- 105 232 331
- CN-U- 203 107 704
- CN-U- 203 677 543
- CN-U- 203 954 197
- CN-U- 205 339 561
- CN-U- 205 339 562
- CN-U- 205 359 992
- CN-U- 205 549 013
- CN-Y- 2 897 247
- JP-A- H08 126 683
- JP-B2- 4 048 337
- US-A- 5 478 337
- US-A1- 2004 199 139

## Description

### FILED OF THE INVENTION

The present invention relates to a medication mixer.

### BACKGROUND OF THE INVENTION

In case of using a penicillin bottle to contain powder injection, freeze-dried powder injection or liquid injection, it needs to draw out liquid medication or water for injection in a soft intravenous bag into the penicillin bottle by an injector, until there is enough liquid medication therein; and then to shake the penicillin bottle repeatedly, till the medication in the bottle is mixed evenly. Next, the liquid medication in the penicillin bottle is extracted out into the soft intravenous bag by the injector, until the liquid medication in the bottle is all extracted.

First, the above-mentioned medication mixing process is relatively time-consuming, and strenuous and too many consumable items are spent, such as injectors. More seriously, in the above-mentioned medication mixing process, it is very easy to inject outdoor air into the penicillin bottle and the soft intravenous bag. Under common conditions, there is plenty of various dusts and germs in the air, which may cause very serious medical negligence after the dusts and germs are mixed into the liquid medication to be injected and then into a human body.

Second, as for the traditional infusion preparation, the injection is performed after the medication preparation. The medication in the penicillin bottle is pumped into the soft intravenous bag in advance, and then the medication-prepared soft intravenous bag is brought to an inpatient ward to carry out infusion to a patient. After the medication preparation, the empty penicillin bottle is put aside. A medical worker has no idea of the medication in the soft intravenous bag, the medication having no traceability. Once the medical worker makes a mistake when preparing the infusion, the consequence is unthinkable.

Third, for some special medications, for example the medication to be used immediately after prepared, the traditional medication preparation is not convenient, and various structures of medication mixers disclosed before the present patent do not solve this problem.

Finally, various structures of medication mixers disclosed before the present patent do not solve the problem of liquid leakage.

For the demand on a higher level of medical service, there is an urgent need of an infusion product which is conveniently, safely, and reliably used and has no safety hazard.

In addition, the medication mixer is welded on the soft intravenous bag. After the soft intravenous bag is filled, it needs to perform high temperature sterilization on the whole soft intravenous bag at a temperature of 115-121 degrees Celsius for 30-15 minutes, with a sterilization pressure of 0.15 MPa. Although the medication mixer and the soft intravenous bag are made of polypropylene which can withstand the temperature of 120 degrees Celsius, the material of polypropylene is inevitably softened. Additionally, the high pressure of 0.15 MPa, equivalent to the pressure of 150N per square centimeter, is fatal for the sealed medication mixing cup. First, at a temperature of 120 degrees Celsius, the medication mixing cup and the sealing membrane may have reduced mechanical strength; second, the pressure of 150N per square centimeter directly deforms the body of the medication mixing cup, stretches the sealing membrane, causes wrinkle, and damages the sealing property and the medication mixing cup.

Prior to the present invention, the terminal sterilization of the medication mixer with a sealed medication mixing cup is insurmountable; and the non-sealed medication mixing cup cannot meet the sterility requirement in use. The terminal sterilization of the soft intravenous bag is necessary and obligatory in terms of laws and regulations as well as practical security.

EP2881101A1 discloses a conventional mixing cup in the prior art.

### SUMMARY OF THE INVENTION

The present invention has an object of proposing a soft intravenous bag which is used conveniently and reliably and has no safety hazard, and its related medication mixer and ports.

The invention is set out in the appended set of claims.

### BRIEF DESCRIPTION OF THE DRAWINGS

Figure 1 is a sectional view of a base and a medication mixing cup;
Figure 2 is a top view of a rubber plug;
Figure 3 is a sectional view of an elastic clamping base;
Figure 4 is a cross needle;
Figure 5 is a cross needle with a side through hole;
Figure 6 is a perspective view of the cross needle with a side through hole;
Figure 7 is a cross needle coated with an elastic scalable film;
Figure 8 is a cross needle with the scalable film compressed;
Figure 9 is a medication mixer mounted with the elastic clamping base and the cross needle;
Figure 10 is a structural diagram of the integrated cross needle and the elastic clamping base;
Figure 11 is a structural diagram of a medication mixer mounted with an integrated elastic clamping base;
Figure 12 is a sectional view of the medication mixing cup provided with the elastic clamping jaw therein;
Figure 13 is a medication mixer provided with the cross needle;
Figure 14 is a structural diagram after a penicillin bottom is put in the medication mixer;
Figure 15 is a medication mixer with an easy-breaking handle;
Figure 16 is a soft intravenous bag with a medication mixer;
Figure 17 is a medication mixing cup with a strengthening structure;
Figure 18 is a cover plate with a strengthening structure;
Figure 19 is a cross needle with a clamping base; and
Figure 20 is a pierced medication mixer.

### Reference numerals:

1, soft intravenous bag, 2, base, 3, medication mixing cup, 3-1, cup wall, 3-2, cup bottom, 3-3, reinforcing rib, 4, elastic clamping base, 4-1, elastic clamping jaw, 4-2, annular protrusion, 4-3, central hole, 4-4, supporting column, 4-5, bottom plate, 4-6, clamping ring, 5, cross needle, 5-1, needle plate, 5-2, upper needle, 5-3, lower needle, 5-4, side hole, 5-5, needle point, 6, rubber plug, 6-1, piercing region, 6-2, convex ring, 6-3, concave groove, 7, medication mixing passage, 8, membrane, 9, penicillin bottle, 10, easy-breaking handle, 11, infusion passage, 12, cover plate, 12-1, through hole, 12-2 reinforcing ribs of the cover plate, 13, sealing membrane.

### DETAILED DESCRIPTION OF THE EMBODIMENTS

In order to make the objectives, technical solution and advantages of the present invention clearer, the present invention is further explained in detail with combination of the embodiments, describing partly claimed subject-matter, partly highlighting specific aspects of the disclosure, and with reference to the drawings. It shall be understood that the descriptions are only illustrative, but not to limit the scope of the present invention, which is defined by the claims. In addition, in the following explanation, the description of the well-known structure and technology is omitted to avoid unnecessarily confusing the concepts of the present invention.

### First embodiment

The present invention will be further explained in combination with drawings of the present invention.

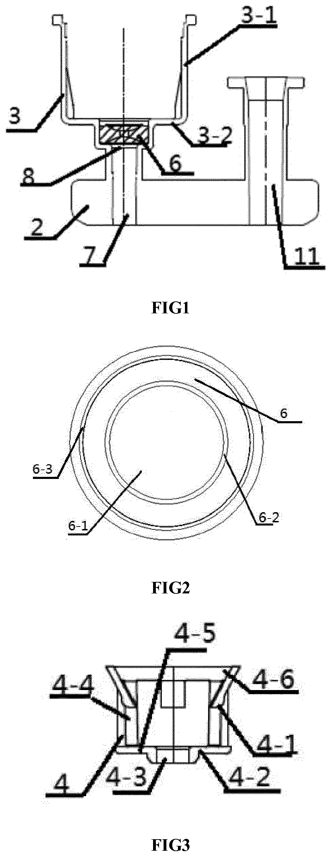

As shown in the sectional view of the base 2 and the medication mixing cup 3 in Figure 1, the medication mixer includes a base 2, a medication mixing cup 3, a rubber plug 6, a medication mixing passage 7, a membrane 8 and an infusion passage, wherein the medication mixing cup 3 consists of a cup wall 3-1 and a cup bottom 3-2; the base 2, the medication mixing cup 3, the membrane 8 and the medication mixing passage 7 integrally form the body structure of the medication mixer.

The rubber plug 6 is placed in the medication mixing passage 7, and is located above the membrane 8.

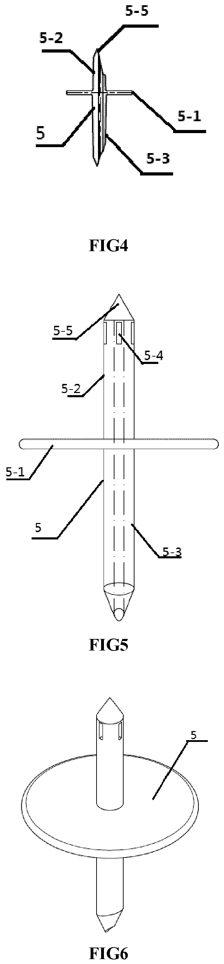

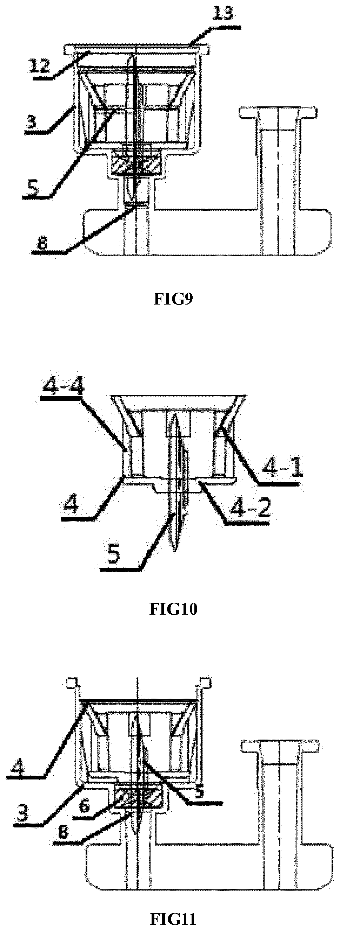

As shown in Figure 11, the medication mixer further includes a cross needle 5, located in the medication mixing cup 3. As shown in Figures 4-8, the cross needle 5 includes a needle plate 5-1, an upper needle 5-2, a lower needle 5-3 and a needle point 5-5, wherein the upper needle 5-2 is integrated with the lower needle 5-3, the needle plate 5-1 combines the upper needle 5-2 with the lower needle 5-3, and the heads of the upper needle 5-2 and the lower needle 5-3 are provided with the needle points 5-5. For the structures of the upper needle 5-2 and the lower needle 5-3 of the cross needle 5, except for the structure of the needle point 5-5 as shown in Figure 5, the side hole 5-4 of the hollow passage of the upper needle 5-2 may be arranged on the side wall of the point of the upper needle 5-2, as shown in Figures 5,6; similarly, the side hole 5-4 of the hollow passage of the lower needle 5-3 is arranged on the side wall of the point of the lower needle 5-3 (not shown). Such an arrangement is to avoid plenty of chippings caused by an edge of the side hole 5-4 directly cutting a rubber plug or a bottle plug when the rubber plug 6 or the bottle plug is pierced by the upper needle 5-2 and the lower needle 5-3. By arranging the side hole 5-4 on the side wall of the needle point, the needle point 5-5 is directly pierced into the rubber plug 6, without direct cutting, which reduces the chippings.

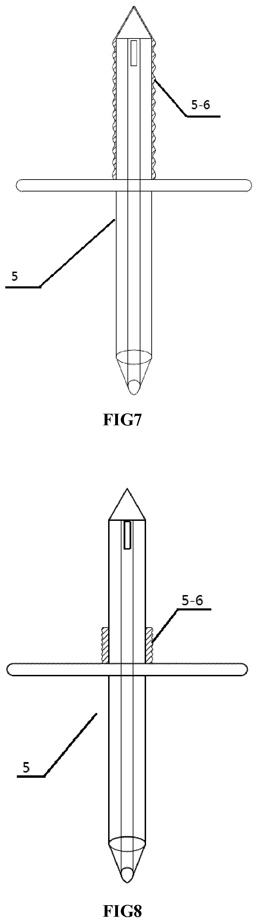

As shown in Figure 7, a layer of elastic shrink film is coated on the upper needle 5-2, and is compressed after being pierced, stacking around a piercing hole, as shown in Figure 8, thereby effectively preventing the liquid medication from leaking out along a gap of the piercing hole. Obviously, the lower needle 5-3 is similarly coated with a layer of elastic shrink film, so as to solve the problem of leakage of the liquid medication along the gap of the piercing hole.

The medication mixing passage 7 penetrates through the base 2, and upwards extends through the cup bottom 3-2 of the medication mixing cup 3. The membrane 8 functions to avoid the direct contact of the liquid medication with the rubber plug 6, so as to ensure the complete sealing before use. Theoretically, the membrane may be located at any position in the medication mixing passage 7 below the rubber plug 6. However, if the position of the membrane 8 is too low, the lower needle 5-3 of the cross needle 5 is too long, so preferably, the membrane 8 is located below the rubber plug 6 and clings to the lower surface of the rubber plug 6.

The rubber plug 6 is placed in the medication mixing passage 7 and above the membrane 8. The rubber plug 6 functions to ensure that the liquid medication does not ooze from the medication mixing passage 7 after flowing out of the gap between the membrane 8 and the outer wall of the lower needle 5-3 after the rubber plug 6 and the membrane 8 are pierced by the lower needle 5-3. That is, the outer diameter of the rubber plug 6 is matched with the inner diameter of the medication mixing passage 7, such that the rubber plug 6 is closely contacted with the medication mixing passage 7.

Preferably, the upper surface of the rubber plug 6 is substantially flushed with or slightly higher than the upper surface of the cup bottom 3-2. Thus, the lower surface of the elastic clamping base 4 as shown in Figure 3 presses the upper surface of the rubber plug 6.

As a preferable technical solution, the membrane 8 is located below the rubber plug 6 and clings to the lower surface of the rubber plug 6, as shown in Figure 1, which may prevent the liquid medication from seeping into the medication mixing passage 7 after the lower needle 5-3 pierces the membrane 8. In particular, a relatively thin piercing region 6-1 defined by the convex ring 6-2 or the concave groove 6-3 is arranged on the surface of the rubber plug 6 as shown in Figure 2. Corresponding to the convex ring 6-2 or the concave groove 6-3 on the rubber plug 6, the concave groove 6-3 or the convex ring 6-2 is arranged on the upper surface of the membrane 8. Obviously, the convex ring 6-2 or the concave groove 6-3 on the rubber plug 6 is tabled with the concave groove 6-3 or the convex ring 6-2 on the membrane 8, thereby better preventing the leakage of the liquid medication. Optionally, the convex ring may also be 6-3, and the concave groove may be 6-2.

After the needle plate 5-1 presses against the rubber plug 6, the rubber plug 6 and the membrane 8 provided with the concave groove 6-3 or the convex ring 6-2 which are tabled with each other can completely avoid the leakage of the liquid medication after the medication mixing.

More preferably, the lower surface of the needle plate 5-1 surrounds the root of the lower needle 5-3, and is provided with a circle of annular protrusion. After the cross needle 5 with the clamping base is mounted in the medication mixing cup, the annular protrusion of the needle plate 5-1 presses against the rubber plug strongly, so as to realize better sealing.

It can be understood that the infusion passage 11 as shown in Figure 11 can be integrated with the above-mentioned medication mixer body, and is arranged on the base 2 parallel with the medication mixer, as shown in Figure 1. Another infusion port can be used when the soft bag is made. For example, the infusion passage can be independently welded on the soft intravenous bag 1 by another base 2, at the same side as the medication mixer or the side opposite to the medication mixer, or other portions of the soft intravenous bag 1, which is obvious for persons skilled in the art.

The medication mixer further includes an elastic clamping base 4, preferable, an independent elastic clamping base 4 as shown in Figure 3. The cross needle 5 is placed between the elastic clamping jaw 4-1 and the bottom plate 4-5. As shown in Figure 3, the elastic clamping base 4 includes one clamping ring 4-6, the lower side of which is provided with the elastic clamping jaw 4-1. The upper ends of the elastic clamping jaw 4-1 are arranged uniformly and fixed along the clamping ring 4-6, and the lower ends of the elastic clamping jaw 4-1 are free ends. The elastic clamping jaw 4-1 inclines towards the center of the medication mixing cup 3 from the fixed upper ends to the free ends. The elastic clamping base has a bottom plate 4-5, a supporting column 4-4, a clamping ring 4-6 and an elastic clamping jaw 4-1 which are integrally formed. The supporting column 4-4 is arranged at the periphery of the bottom plate 4-5, for supporting and fixing the clamping ring 4-6.

The bottom plate 4-5 is provided with a central hole 4-3, and the lower needle 5-3 penetrates through the central hole 4-3, thereby positioning the cross needle. A circle of annular protrusion 4-2 is arranged around the central hole 4-3; the region defined by the annular protrusion 4-2 is completely superimposed with the central hole 4-3, and is axially arranged with the medication mixing passage 7; the bottom plate 4-5 clings to the cup bottom 3-2 by the annular protrusion 4-2. After the elastic clamping base 4 is mounted in the medication mixing cup 3, the annular protrusion 4-2 on the elastic clamping base 4 presses against the rubber plug 6.

The needle plate 5-1 of the cross needle 5 as shown in Figures 4-8 is mounted below the elastic clamping jaw 4-1 in the medication mixing cup 3 and above the bottom plate 4-5. The cross needle 5 includes the hollow upper needle 5-2 and lower needle 5-3 which are integrated as well as a needle plate 5-1 for integrating the upper needle 5-2 and the lower needle 5-3, and the heads of the upper needle 5-2 and the lower needle 5-3 are provided with needle points 5-5.

As for the combination of the cross needle 5, the elastic clamping base 4 as well as the medication mixing cup 3 and the base 2, as shown in Figure 11, the medication mixing cup 3 is integrated with the base 2, the elastic clamping base 4 is placed in the medication mixing cup 3, and the cross needle 5 is clamped in the elastic clamping base 4.

The cup wall is provided with the limit structure (not shown), the cross needle 5 with the clamping base is mounted in the medication mixing cup 3 and is limited by the limit structure. In particular, after the lower needle 5-3 pierces the rubber plug 6 and the membrane 8, the cross needle is completely limited by the limit structure.

In the embodiment, the elastic clamping base 4 is placed in the medication mixing cup 3, and then is limited by the limit structure (not shown) on the inner wall of the cup wall 3-1, thereby preventing the elastic clamping base 4 from sliding out of the medication mixing cup 3 after put in place. In addition, the position of limiting the elastic clamping base 4 makes the bottom of the elastic clamping base 4 contact with the cup bottom 3-2 of the medication mixing cup 3. In the preferable arrangement solution of the rubber plug 6, the annular protrusion 4-2 at the bottom of the elastic clamping base 4 presses against the rubber plug 6.

The needle plate 5-1 of the cross needle 5 mounted in the elastic clamping base 4 is located between the elastic clamping jaw 4-1 and the bottom plate 4-5, and afterwards, the lower needle 5-3 does not pierce the rubber plug 6.

Of course, it can be understood that before use, the lower needle 5-3 partially pierces the rubber plug 6, or as shown in Figure 9, the lower needle 5-3 completely pierces the rubber plug 6, or more preferably, a small hole is arranged at the center of the rubber plug 6 in advance, so as to form an annular rubber plug 6, and the lower needle 5-3 pierces the rubber plug 6 via the small hole, which are all feasible. When the annular rubber plug 6 is designed, the diameter of the small central hole of the rubber plug 6 is less than that of the lower needle 5-3, such that the lower needle 5-3 does not cut the rubber plug 6 after the lower needle 5-3 is inserted in the small hole of the rubber plug 6 and penetrates through the rubber plug 6, thereby effectively avoiding the pollution of the chippings to the liquid medication when the lower needle 5-3 pierces the rubber plug 6. Moreover, since the diameter of the small hole is less than the outer diameter of the lower needle 5-3, the rubber plug 6 still clings to the lower needle 5-3, thereby avoiding the liquid leakage.

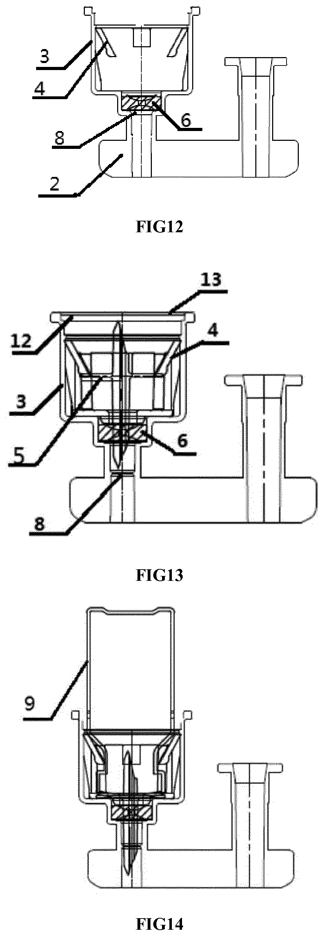

Of course, before the medication mixer is assembled and used, the cross needle 5 is just placed in the medication mixing cup 3, and does not pierce the membrane 8. The rubber plug 6 can be not pierced, or partially pierced, or completely pierced as long as the membrane 8 is not pierced, and the use of the medication mixer is not affected. When the sealing membrane 13 is torn, a partition plate 12 is taken off and the penicillin bottle 9 is inserted, as shown in Figure 14, the bottle neck of the penicillin bottle 9 is pushed down below the elastic clamping jaw 4-1. At this point, the upper needle 5-2 and the lower needle 5-3 of the cross needle 5 respectively pierce the rubber plug 6, the membrane 8 and the bottle plug of the penicillin bottle 9. Simultaneously, the cross needle 5 is completely limited by the limit protrusion on the inner wall of the medication mixing cup 3, that is by the elastic clamping jaw 4-1. The bottle neck of the penicillin bottle 9 is clamped by the elastic clamping jaw 4-1 and cannot withdraw.

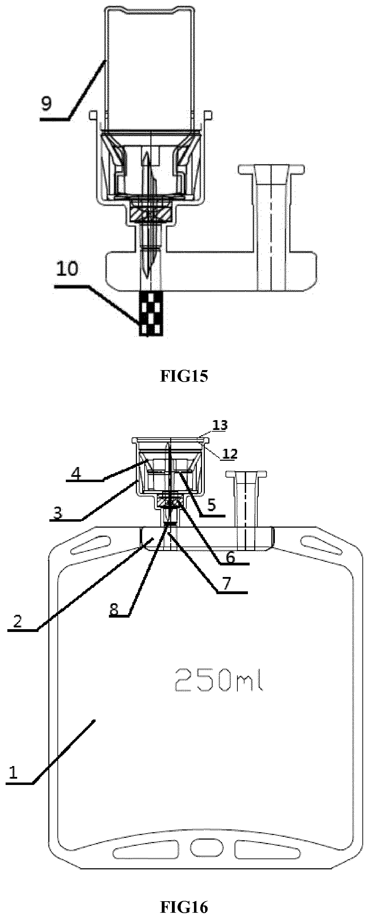

As for the use state, as shown in Figures 14 and 15, the penicillin bottle 9 is inserted in the medication mixing cup 3, and the bottle cap of the penicillin bottle 9 is pressed in the elastic clamping base 4, such that the free end of the elastic clamping jaw 4-1 is located at the bottle neck of the penicillin bottle 9 and is clamped at the bottle neck, thereby clamping the penicillin bottle 9 and preventing the penicillin bottle 9 from withdrawing from the medication mixing cup 3.

Simultaneously, the bottle cap of the penicillin bottle 9 is pierced by the upper needle 5-2, and the bottle cap presses the needle plate 5-1 downwards, thereby pushing the lower needle 5-3 to pierce the rubber plug 6 and then the membrane 8, such that the medication mixing passage 7 is communicated with the penicillin bottle 9 via the cross needle 5.

Obviously, the clamping ring 4-6 of the elastic clamping base 4 has an inner diameter substantially the same as or slightly greater than the outer diameter of the penicillin bottle 9. In order to better fix and limit the penicillin bottle 9 so that the penicillin bottle 9 is not slidable, the cap thickness of the penicillin bottle 9 shall be substantially the same as the distance of the free ends of the elastic clamping jaw 4-1 to the bottom plate 4-5, such that the penicillin bottle 9 is just clamped between the free ends of the elastic clamping jaw 4-1 and the bottom plate 4-5 through the bottle cap, and cannot move up and down.

Of course, it can be understood that for the sake of a machining tolerance, the distance of the free end of the elastic clamping jaw 4-1 to the bottle plate 4-5 is slightly greater than the thickness of the bottle cap, which does not influence the butt joint and fixation of the penicillin bottle 9.

After the cross needle 5, the elastic clamping base 4 and the medication mixing cup 3 are assembled, as shown in Figure 9, there is one preferable solution that the upper surface of the rubber plug 6 is substantially flushed with or slightly higher than the upper surface of the cup bottom 3-2. As such, the lower surface of the elastic clamping base 4 as shown in Figure 3 presses against the upper surface of the rubber plug 6.

The elastic clamping base 4 as shown in Figure 3 is placed in the medication mixing cup 3. The cross needle 5 as shown in Figures 4-8 is placed in the elastic clamping base 4, thereby forming the medication mixer as shown in Figure 9. After the elastic clamping base 4 and the cross needle 5 are mounted, the cover plate 12 is placed at the cup opening of the medication mixing cup 3, and then the medication mixing cup 3 is covered and sealed by the sealing membrane 13.

As shown in Figure 1, the medication mixer, together with the infusion passage 11 arranged on the base 2 parallel with the medication mixer, forms the dual hard ports.

The medication mixer is connected with the soft intravenous bag 1 in a welding way, to form the soft intravenous bag 1 with the medication mixer, as shown in Figure 16. The medication mixer is jointed with the soft intravenous bag 1 by the base 2.

It can be understood that the infusion passage 11 can be arranged on the base 2, parallel with the medication mixer, as shown in Figure 1. The infusion passage 11 can be independently welded on the soft intravenous bag 1 by another base 2, at the same side as the medication mixer or the side opposite to the medication mixer, or other portions of the soft intravenous bag 1, which is obvious for persons skilled in the art.

Finally, in order to ensure the safety and sterility of the soft intraveous bag 1 in use, the medication mixing cup 3 needs to be sealed. The easy-to-tear film is usually used to seal the medication mixing cup 3, preferably, a pp easy-to-tear film, and more preferably, a breathable pp easy-to-tear film. The easy-to-tear film is directly pressed at the cup opening of the medication mixing cup 3, and is directly torn when the medication mixing cup 3 is used.

Since the soft intravenous bag 1 is sealed and then sterilized at a high temperature after filled, if the medication mixing cup 3 is not sealed, the sterilized soft intravenous bag 1 with the medication mixer is inevitably polluted during transportation and use and its sterility cannot be guaranteed due to the cross needle 5 for piercing in the medication mixing cup 3 still exposing in the external environment.

As for the sealed medication mixing cup 3, in sterilization, due to the high temperature and the high pressure, the cup body of the medication mixing cup 3 made of the medical polymer material and the easy-to-tear film of the sealed medication mixing cup 3 will soften and shrink; there is a relatively large pressure difference between the inside and outside of the sealed medication mixing cup 3; under the high pressure, the medication mixing cup 3 and the easy-to-tear film will be deformed, and be crushed after cooled, thereby damaging the structure of the medication mixing cup, and causing the medication mixer not to be used.

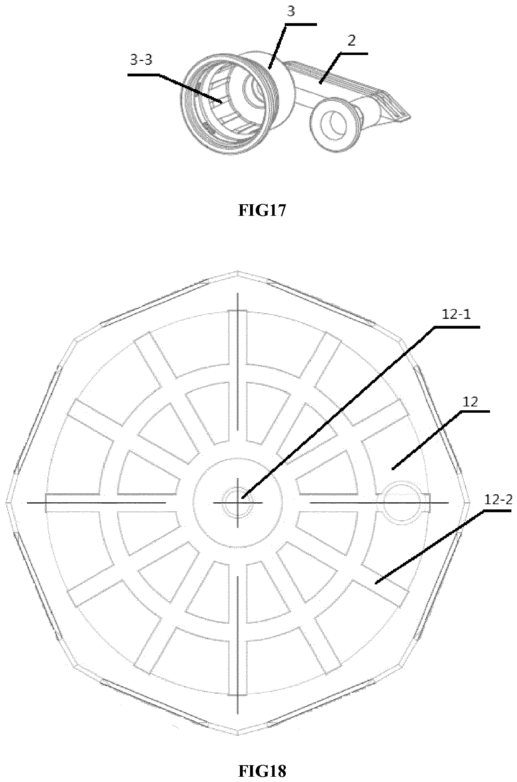

In order to solve the above-mentioned problem, more preferably, the reinforcing ribs 3-3 are arranged on the cup wall 3-1 of the medication mixing cup 3, preferably, at the lower half portion of the cup wall 3-1, as shown in Figure 17.

The reinforcing ribs 3-3 may be protruding vertical bars or horizontal bars which are integrated with the medication mixing cup 3 and uniformly arranged at the inner side and/or outer side of the cup wall 3-1, or may have a criss-crossed net structure. Preferably, the vertical bars are uniformly arranged at the lower half portion of the inner side of the cup wall 3-1; more preferably, the protruding vertical bars extend to the cup bottom 3-2 from the lower half portion of the inner side of the cup wall 3-1, and most preferably, the thickness of the vertical protrusion continuously increases gradually and smoothly from top to bottom.

The arrangement of the reinforcing ribs 3-3 on the cup wall 3-1 may substantially solve the problem that the medication mixing cup 3 is pressed and deformed during the sterilization. However, there still exists the problem that the sealing membrane 13 of the sealed medication mixing cup 3 is compressed and deformed when sterilized.

In order to solve this problem, the cover plate 12 as shown in Figure 18 is further arranged at the cup opening of the medication mixing cup 3, and is covered by the sealing membrane 13, such that the cover plate 12 effectively supports the sealing membrane 13, so as to prevent the sealing membrane 13 from being compressed and deformed in sterilization. The inner stepwise edge is arranged at the cup opening along the cup wall 3-1, and the cover plate 12 is placed on the inner stepwise edge, such that the upper surface of the cover plate 12 arranged at the cup opening is flushed with or slightly lower than the cup opening, which does not influence the sealing process of press welding the sealing membrane 13 on the upper end surface of the cup wall 3-1.

As for the arrangement of the cover plate 12, preferably, the cover plate 12 is arranged coaxially with the cross needle 5, and the needle point 5-5 of the upper needle 5-2 is dead against the center of the cover plate 12.

The cover plate 12 is preferably designed into a polygon with a hole in the center. The experiment shows that after the pressure balancing means is adopted, such a preferable design can balance the pressure inside and outside the medication mixing cup 3 more rapidly, thereby functioning very well in the moist heat sterilization process---the sealing membrane 13 is smooth as before, and the medication mixing cup 3 is not deformed. This is because the polygonal cover plate 12 with a central hole can increase the gas exchanging speed below and above the cover plate 12, and balance the air pressure in the medication mixing cup 3 more rapidly, as well as the air pressure outside and inside the medication mixing cup 3.

With the above-mentioned arrangement, the medication preparation and addition can be performed safely, conveniently and rapidly, which completely solves the problem of secondary pollution at the stage of the medication preparation. Simultaneously, the infusion can be traced since the penicillin bottle 9 cannot be taken out non-destructively after connected and fixed onto the medication mixing cup 3 and clamped by the elastic clamping jaw 4-1. That is, from the medication preparation to the completion of infusion, until the recovery, the medication added and injected can be traced.

However, for the medication to be preserved specially, for example the medication to be used immediately after prepared, the above-mentioned medication mixer may have some inconveniences since the medication filled in the penicillin bottle 9 must be brought to the impatient ward, then the penicillin bottle 9 is abutted with the medication mixer, and the medication can be used after preparation in the ward.

In order to meet the requirement of using the medication immediately after prepared, on the basis of the above-mentioned structure, the easy-breaking handle 10 is additionally arranged, as shown in Figure 15.

The easy-breaking handle 10 is arranged at the lower end of the medication mixing passage 7. As shown in Figure 15, having abutted with the elastic clamping base 4 in the medication mixing cup 3 and is pierced, the penicillin bottle 9 is brought to the ward. Before infusion, the easy-breaking handle 10 is broken, the medication mixing passage 7 is broken through, and the on-the-spot medication preparation and use can be realized.

As for the shape and structure of the base 2, in order to avoid the damage of the welding portion of the base 2 to the soft intravenous bag 1 in the processes of storing, transporting and using after the medication mixer, the infusion passage 11 or the dual hard ports with the medication mixer and the infusion passage 11 are welded on the soft bag, the base 2 is designed into a shape of a dumbbell or ship, as shown in Figure 1, and the lower ends of the medication mixing passage 7 and the infusion passage 11 are flushed with the lower end of the base 2. The welding lines are uniformly distributed on the side wall all around the base 2, which may ensure that the base 2 may be well fused and welded with the soft intravenous bag 1 even at a relatively low temperature in the welding process of the soft intravenous bag 1. Moreover, the streamlined base 2 of a shape of ship or dumbbell not only improves mechanical property of welding, but also makes the combination of the dual hard ports with the soft intravenous bag 1 more smooth without a sharp angle, and does not tend to damage the soft bag.

As for the selection of the materials of the medication mixer and the soft intravenous bag 1, the soft intravenous bag 1 is made of the widely used non-PVC officinal compounding velamen, including three or five layers.

The medication mixer is made of the medical polypropylene material having good compatibility with the non-PVC material of the soft intravenous bag 1. The base 2, the medication mixing cup 3 and the cover plate 12 are preferably made of the polypropylene R530C material; however, the cross needle 5 and the clamping body are preferably made of the P17 material in the pp material system in view of its piercing property and mechanical characteristics.

The cross needle 5, the limit protrusion and the limit structure are made of the polypropylene material, preferably, the polypropylene P17 material.

Finally, the most critical point of the medication mixer with a sealing structure is the terminal sterilization after the sealed medication mixer is welded on the soft bag. Currently, from the point of view of laws and regulations as well as practical injection safety, the terminal sterilization must be performed to all the medication packages before the filling and delivery.

At present, there are mainly two sterility assurance processes for the injection:
1. A process of terminal sterilization: on the basis of controlling a pollution load of microorganism, after the medication is filled, the degerming is realized by moist heat sterilization. Usually, this method has a low cost, a high level of sterility assurance, and is suitable for sterilizing both a large volume injection and a small volume injection.
2. A process of sterile production: under an environment of a sterile system, by sterile filtration or sterile operation, for the purpose of de-pollution, the sterility level is assured by eliminating various possibilities of causing pollutions. Generally, due to a high demand of this method on the environment system, and many factors of influencing the sterile operation, the sterility assurance level is lower than that in the terminal sterilization process. The sterile production process is usually suitable for powder-injection, and also for clinical needs, but not for the small volume injector which may be realized in the terminal sterilization. Thus, the terminal sterilization process has different system requirements, different sterilization methods and different sterilization assurance results from the sterile production process.

The large volume infusion is very sensitive to the cost. Therefore, the terminal sterilization in the large volume infusion may only adopt the moist heat sterilization process with a low cost and high efficiency, which is usually conducted at a high temperature of 115-121 degrees Celsius, under the steam with a pressure of 0.15 MPa, for 30-15 minutes.

Although the medication mixer and the soft intravenous bag 1 are made of the polypropylene material withstanding the high temperature of 120 degrees Celsius, at such a high temperature, the sealed medication mixer will degrade in mechanical characteristics, and tends to deform under the intensity of pressure of 0.15 MPa; however, the sealing membrane 13 will also be deformed and wrinkled at this temperature and intensity of pressure, losing the sealing effect.

From the year 2011 to 2014, hundreds of experiments were conducted, and the solution is determined from different aspects.

First, as for the structure of the medication mixing cup 3, as shown in Figure 17, the reinforcing ribs 3-3 are arranged at the lower half portion of the cup body. The reinforcing ribs 3-3 are arranged at the cup wall 3-1 of the medication mixing cup 3, preferably, at the lower half portion of the cup wall 3-1.

The reinforcing ribs 3-3 may be protruding vertical bars or horizontal bars which are integrated with the medication mixing cup 3 and uniformly arranged at the inner side and/or outer side of the cup wall 3-1, or may have a criss-crossed net structure. Preferably, the vertical bars are uniformly arranged at the lower half portion of the inner side of the cup wall 3-1; more preferably, the protruding vertical bars extend to the cup bottom 3-2 from the lower half portion of the inner side of the cup wall 3-1, and most preferably, the thickness of the vertical protrusion continuously increases gradually and smoothly from top to bottom.

The medication mixing cup 3 with the reinforcing ribs 3-3 improves the mechanical pressure resistance of the cup body to a considerable extent. With the experimental comparison, the medication mixing cup 3 without the reinforcing ribs 3-3 is compressed into a square from the initial circular shape after the moist heat sterilization is performed, and cannot be used any more.

After the reinforcing ribs 3-3 are arranged, subsequent to the moist heat sterilization process, the circular medication mixing cup body is slightly compressed, and can be continue to use normally.

Moreover, the sealing membrane 13 is only a very thin easy-to-tear film, for sealing the medication mixing cup 3 and being convenient to tear out in use. The sealing membrane 13 is thinner than the cup body of the medication mixing cup 3 since the sealing membrane 13 itself is only a layer of thin film with a thickness of micron dimension, and cannot withstand the intensity of pressure of 0.15 MPa in the moist heat sterilization process.

As for this, numerous experiments show that the cover plate 12 as shown in Figure 13 is arranged under the sealing membrane 13, and is covered by the sealing membrane 13, such that the cover plate 12 effectively supports the sealing membrane 13, so as to prevent the sealing membrane 13 from being compressed and deformed in sterilization. The cover plate 12 is placed on an inner stepwise edge of the cup opening of the medication mixing cup 3, such that the upper surface of the cover plate 12 arranged at the cup opening is flushed with or slightly lower than the cup opening. Such an arrangement of the cover plate 12 does not influence the sealing process of press welding the sealing membrane 13 on the upper end surface of the cup wall 3-1.

The cover plate 12 has a circular shape substantially matched with the shape of the cup opening of the medication mixing cup 3. Preferably, the cover plate 12 has a shape in cross section of polygon, for example, pentagon, hexagon, octagon and dodecagon. The polygonal cover plate 12 is conveniently taken out in use; its another unexpected effect will be mentioned in the following.

In addition, in order to enhance the compressive strength of the cover plate 12, preferably, the cover plate 12 is arranged coaxially with the cross needle 5, with the needle point 5-5 of the upper needle 5-2 dead against the center of the cover plate 12, for supporting the cover plate 12. More preferably, one through hole 12-1 is arranged in the center of the cover plate 12 which has an inner diameter less than an outer diameter of the upper needle 5-2. The needle point 5-5 of the upper needle 5-2 is partially located in the through hole 12-1, but cannot penetrate therethrough. That is, the needle point 5-5 of the upper needle 5-2 is embedded in the through hole 12-1 of the cover plate 12, thereby better supporting the cover plate 12 by the upper needle 5-2.

Simultaneously, in view of an intense downward pressure born by the cover plate 12, the cover plate 12 is reinforced other than the design of supporting the cover plate 12 by the upper needle 5-2. The radial and annular reinforce ribs 12-2 as shown in Figure 18 enhance the mechanical strength of the cover plate 12.

The sealing membrane 13 press welded on the upper end of the cup opening is tightly stuck on the upper surface of the cover plate 12, such that the sealing membrane 13 does not need to withstand a high pressure, which greatly buffer the deformation and wrinkle of the sealing membrane 13.

As for the wrinkle, since the sealing membrane 13 is too thin, the wrinkle will be caused even by a slight pressure, which seriously influences the vision effect of the product.

By many contrast experiments, a layer of thin metal film is plated on the upper surface of the sealing membrane 13 may effectively alleviate the problem of wrinkle of the sealing membrane.

Although the sealed medication mixer with such an arrangement withstands the high temperature and high pressure in the moist heat sterilization process and can be used normally, the product appearance cannot be kept in a good state. In view of the infusion product, it is not qualified.

The above-mentioned design for the structure of the medication mixer can only ensure the use function. In order to completely solve the problem of terminal sterilization of the sealed medication mixer, it needs to fundamentally solve the balance of air pressure inside and outside in the moist heat sterilization of the sealed medication mixing cup.

After the medication mixing cup 3 is assembled and before the cover plate 12 and the sealing membrane 13 are mounted to seal the medication mixing cup, a certain amount of liquid is prefilled in the cup body, and then the cover plate 12 is mounted to seal the medication mixing cup.

When the terminal sterilization is performed on the sealed medication mixing cup 3 with liquid filled in and the soft intravenous bag 1, the liquid is rapidly vaporized at a high temperature, thereby balancing the pressure inside and outside the cup body rapidly.

As for the prefilled liquid, preferably, the thermal capacity is relatively small, to saturate the liquid with a relatively high steam pressure. We have studied that the states of various liquid at a temperature of 120 degrees Celsius under the pressure of 0.15MPa, including common harmless liquid such as water and ethyl alcohol, can meet our requirements. In view of costs and safety, preferably, the prefilled liquid is water.

The important prefilled water amount V0 can be confirmed through the following equation:
PV = nRT, wherein P is an inside-outside pressure difference in the moist heat sterilization, V is a volume of the medication mixing cup 3, n is a mole number of the prefilled liquid/water, R is a gas constant, and T is an absolute temperature in the moist heat sterilization.

V0 = n^{∗}M/ρ, wherein M is a mole mass of the liquid/water, and ρ is a density of the liquid/water.

According to the above-mentioned equation, the pressure inside and outside in the terminal sterilization of the sealed medication mixer can be well balanced.

It is a preferable solution that the pressure inside and outside is balanced in a manner of prefilled liquid.

However, the above-mentioned solution may solve the problem of pressure balance. In practical use, there still exist some problems. The most typical problem is that the finished soft intravenous bag subjected to sterilization, after being cooled, has water drops or liquid in the medication mixing cup, which affects the impression. Moreover, such a product is not accepted by hospitals or patients.

On this basis, one more preferably solution is that we have specially studied the breathable sealing membrane 13 based on the pp material system, which ensures sufficient gas exchange of the breathable sealing membrane 13 with outside after the vaporization of liquid, so there is no residual liquid in the medication mixing cup 3 after the sterilization.

Through many experiments for a long time, the air permeability of the breathable sealing membrane 13 is, most preferably, 5%-35%.

As for the medication mixer sealed by our breathable sealing membrane 13, subsequent to the moist heat high temperature sterilization, the medication mixing cup 3 is not deformed, and the sealing membrane 13 is smooth as before, without any wrinkle.

Of course, there is one improved solution that the breathable sealing membrane 13 with a suitable air permeability is directly adopted to seal the medication mixer, without pre-filling liquid, which is also feasible, proved by many experiments.

After the assembled medication mixer, that is the cross needle 5 with the clamping base, is placed in the medication mixing cup 3, the cup opening of the medication mixing cup 3 is sealed usually by the sealing membrane 13 or the easy-to-tear sealing membrane in a press welding way; or one cover plate 12 is placed at the cup opening, and then the sealing membrane 13 is placed thereon. The sealed medication mixing cup 3 is welded on the soft intravenous bag 1, so as to form the soft intravenous bag 1 with the medication mixer, as shown in Figure 16. Subsequent to the terminal sterilization, the mixer is ready for medical workers to use.

### Second embodiment (not yet claimed)

On the basis of the above-mentioned first embodiment, as for the arrangement of the elastic clamping base 4 in the first embodiment, in the second embodiment as shown in Figure 12, the elastic clamping base 4 in the first embodiment is substituted with the limit protrusion direclty fixed on the inner wall of the medication mixing cup 3. To be specific, the limit protrusion (not shown) is arranged on the inner wall of the cup wall 3-1, and the cross needle 5 with the clamping base is mounted in the medication mixing cup 3 and is limited by the limit protrusion. In particular, after the lower needle 5-3 as shown in Figure 5 pierces the rubber plug 6 and the membrane 8, the cross needle 5 is completely limited by the limit protrusion. As for the limit protrusion, preferably, the elastic clamping jaw 4-1 is used; more preferably, the elastic clamping jaw 4-1 as the limit protrusion is integrated with the medication mixing cup 3. The upper ends of the elastic clamping jaw 4-1 are arranged evenly and fixed along the periphery of the inner wall of the cup wall 3-1. The lower ends of the elastic clamping jaw are free ends, and the elastic clamping jaw 4-1 inclines towards the center of the medication mixing cup 3 from the fixed upper ends to the free ends, as shown in Figure 12.

After the cross needle 5 as shown in Figures 4-8 is mounted in the medication mixing cup 3 with the limit protrusion, as shown in Figure 13, the needle plate 5-1 of the cross needle 5 is limited between the limit protrusion and the cup bottom 3-2 by the limit protrusion and cannot be taken out. Figure 13 shows the state that the cross needle 5 has pierced the rubber plug 6 but does not pierce the membrane 8.

Obviously, the distance of the upper surface of the cup bottom 3-2 to the lower surface of the membrane 8 is less than the length of the lower needle 5-3.

Other structures of the medication mixer are the same as those in the first embodiment.

### Third embodiment

On the basis of the above-mentioned first embodiment, as for the arrangement of the elastic clamping base 4 in the first embodiment, in the third embodiment as shown in Figure 10, the cross needle 5 is directed fixed with the elastic clamping base 4, and preferably, the cross needle 5 is integrated with the elastic clamping base 4.

Like the elastic clamping base 4 in the first embodiment, the elastic clamping base 4 in the third embodiment includes one clamping ring 4-6 provided with an elastic clamping jaw 4-1 at the lower side. The upper ends of the elastic clamping jaw 4-1 are arranged uniformly and fixed along the clamping ring 4-6, and the lower ends of the elastic clamping jaw 4-1 are free ends. The elastic clamping jaw 4-1 inclines towards the center of the medication mixing cup 3 from the fixed upper ends to the free ends. The elastic clamping base 4 has a bottom plate 4-5, a supporting column 4-4, a clamping ring 4-6 and an elastic clamping jaw 4-1 which are integrally formed. The supporting column 4-4 is arranged at the periphery of the bottom plate 4-5, for supporting and fixing the clamping ring 4-6.

The difference between the first embodiment and the third embodiment is that the central hole 4-3 on the bottom plate 4-5 is omitted, and the cross needle 5 is integrally fixed on the bottom plate 4-5 directly. The structures of the upper needle 5-2 and the lower needle 5-3 are the same as those as shown in Figures 4-8. The hollow passages of the upper needle 5-2 and the lower needle 5-3 penetrate through the center of the bottom plate 4-5 of the elastic clamping base 4 and are communicated with each other.

A circle of annular protrusion 4-2 is arranged at the root of the lower needle 5-3. After the elastic clamping base 4 is mounted in the medication mixing cup 3, the annular protrusion 4-2 on the elastic clamping base 4 presses against the rubber plug 6.

Before use, the elastic clamping base 4 is mounted in the medication mixing cup 3 and is limited by the cup wall 3-1 and cannot be taken out when pushed to the cup bottom 3-2. As shown in Figure 11, in the case that the elastic clamping base 4 with the cross needle 5 is pushed to the cup bottom 3-2, the lower needle 5-3 pierces both the rubber plug 6 and the membrane 8.

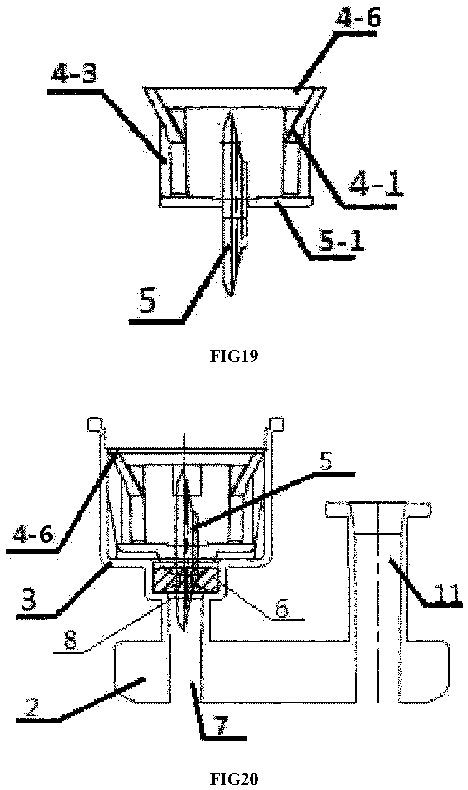

Of course, or, as shown in Figure 19, the supporting column 4-3, the clamping ring 4-6 and the elastic clamping jaw 4-1 are integrally arranged on the needle plate 5-1, and the supporting column 4-3 is arranged at a periphery of the needle plate 5-1 to support and fix the clamping ring 4-6.

The upper ends of the elastic clamping jaw 4-1 are arranged evenly and fixed along the clamping ring. The lower ends of the elastic clamping jaw are free ends, and the elastic clamping jaw 4-1 inclines towards the center of the medication mixing cup from the fixed upper ends to the free lower ends. There is a plurality of elastic clamping jaws 4-1, preferably, three or four.

The limit protrusion is arranged on an inner wall of the cup wall. After the cross needle is mounted in the medication mixing cup, the needle plate is limited between the limit protrusion and the cup bottom by the limit protrusion, and cannot be taken out. The limit protrusion herein is preferably an elastic clamping jaw.

The cross needle 5 with a clamping base is placed in the medication mixing cup 3. As shown in Figure 20, the cross needle 5 with the clamping base placed in the medication mixing cup 3 is pushed to the cup bottom 3-2, thereby piercing the rubber plug 6 and the membrane 8.

Other structures of the medication mixer are the same as those in the first embodiment.

It shall be understood that the above embodiments of the present invention are only used for illustratively explaining the principle of the present invention, without limiting the present invention.

## Claims

1. A medication mixer, comprising a base (2), a medication mixing passage (7) and a medication mixing cup (3) which are integrally formed, and a cross needle (5), wherein the medication mixing cup consists of a cup wall (3-1) and a cup bottom (3-2);
the cross needle consists of an integrated needle plate (5-1) as well as an upper needle (5-2) and a lower needle (5-3) with hollow passages which are communicated with each other;
wherein
the lower end of the medication mixing passage (7) penetrates through the base (2), with the upper end penetrating through the cup bottom, a separating membrane (8) is arranged in the medication mixing passage (7) between the base (2) and the cup bottom (3-2), and a rubber plug (6) is arranged in the medication mixing passage (7) between the separating membrane (8) and the cup bottom (3-2);
the distance of the upper surface of the cup bottom (3-2) to the lower surface of the separating membrane (8) is less than the length of the lower needle (5-3);
the medication mixer further comprises a sealing membrane (13) connected at a cup opening of the medication mixing cup in a press welding way, so as to seal the medication mixing cup;
the sealing membrane (13) is an easy-to-tear membrane which can still be basically kept smooth after moist heat terminal sterilization;
**characterized in that**
further comprising an elastic clamping base (4) including a clamping ring (4-6) provided with an elastic clamping jaw (4-1) at the lower side, the upper ends of the elastic clamping jaw (4-1) are arranged uniformly and fixed along the clamping ring, and the lower ends of the elastic clamping jaw are free ends, and the elastic clamping jaw (4-1) inclines towards the center of the medication mixing cup from the fixed upper ends to the free lower ends;
the needle plate (5) is mounted below the elastic clamping jaw (4-1) in the medication mixing cup;
a limit structure is arranged on an inner wall of the cup wall, the elastic clamping base (4) is mounted in the medication mixing cup (3) and is limited by the limit structure;
the medication mixing cup (3) is prefilled with a certain amount of liquid before sealed by the sealing membrane (13), for substantially balancing the air pressure inside and outside the medication mixing cup;
the liquid can be vaporized rapidly in an environment of moist heat sterilization.

2. The medication mixer according to claim 1, wherein
the elastic clamping base (4) has a bottom plate (4-5), a supporting column (4-4), a clamping ring (4-6) and an elastic clamping jaw (4-1) which are integrally formed, wherein the supporting column (4-4) is arranged at a periphery of the bottom plate (4-5), so as to support and fix the clamping ring (4-6);
a central hole is arranged on the bottom plate (4-5), the lower needle penetrates through the central hole, thereby positioning the cross needle;
the needle plate (5-1) is located between the bottom plate (4-5) and the elastic clamping jaw (4-1).

3. A medication mixer with a strengthening structure, comprising the medication mixer according to any one of claims 1-2;
reinforcing ribs (3-3) are arranged on the cup wall (3-1) of the medication mixing cup (3), so as to enhance compressive strength of the cup wall;
the reinforcing ribs (3-3) are integrally arranged at an inner side of the cup wall (3-1) in an up and down direction and/or a horizontal direction.

4. The medication mixer according to claim 3, wherein
a piercing region is formed in the central region of the rubber plug (6), and is thinner than an edge region;
an annular protrusion is arranged at the central region of the lower surface of the needle plate (5-1), and the piercing region is superimposed with or located in the region limited by the annular protrusion.

5. The medication mixer according to claim 3, wherein
the medication mixer further includes a cover plate (12); the cover plate is located in the cup opening;
an inner stepwise edge is arranged along the cup wall at the cup opening, the cover plate is put on the inner edge;
the upper surface of the cover plate (12) is flushed with or slightly lower than the cup opening;
the sealing membrane (13) is hot-pressed on the upper end surface of the cup wall, thereby sealing the cup opening;
the cover plate is polygonal, with a hole (12-1) in the center, and the point of the upper needle is located in the hole and does not penetrate therethrough.

6. The medication mixer according to any one of claims 3-5, wherein
the sealing membrane is a breathable easy-to-tear membrane with an air permeability of 5% to 35%;
a metal film is plated on an upper surface of the sealing membrane.

7. The medication mixer according to claim 3, wherein
the lower end of the medication mixing passage is provided with an easy-breaking handle for sealing the lower end of the medication mixing passage.

8. The medication mixer according to claim 3, wherein
a convex ring or a concave groove is arranged on the upper surface of the separating membrane (8), a concave groove or a convex ring is arranged correspondingly on the lower surface of the rubber plug (6);
the separating membrane (8) is tabled with the rubber plug (6) through the convex ring or the concave groove.

9. The medication mixer according to claim 3, wherein
an exit of the hollow passage of the upper needle is arranged on the side wall of the point of the upper needle (5-2);
and/or,
an exit of the hollow passage of the lower needle is arranged on the side wall of the point of the lower needle (5-3);
the upper needle is coated with a layer of elastic shrink film;
and/or,
the lower needle is coated with a layer of elastic shrink film.

10. Dual hard ports with a medication mixer, comprising the medication mixer according to any one of claims 1-9, wherein
one infusion passage is arranged on the base of the medication mixer at the side opposite to the medication mixing passage; and
ports are arranged on the infusion passage.

11. A soft intravenous bag, comprising the medication mixer according to any one of claims 1-9;
or comprising the dual hard ports according to claim 10;
the medication mixer or the dual hard ports jointed with the soft bag by the base;
further comprising an infusion port which is arranged on the soft intravenous bag at the same side as the medication mixer, or arranged on the soft intravenous bag at the side opposite to the medication mixer.

## Patentansprüche

1. Medikamentenmischer, umfassend eine Basis (2), einen Medikamentenmischkanal (7) und einen Medikamentenmischbecher (3), die einstückig ausgebildet sind, und eine Kreuznadel (5), wobei der Medikamentenmischbecher aus einer Becherwand (3-1) und einem Becherboden (3-2) besteht;
wobei die Kreuznadel aus einer integrierten Nadelplatte (5-1) sowie einer oberen Nadel (5-2) und einer unteren Nadel (5-3) mit hohlen Durchgängen, die miteinander in Verbindung stehen; besteht,
wobei das untere Ende des Medikamentenmischkanals (7) die Basis (2) und das obere Ende den Becherboden durchdringt, wobei im Medikamentenmischkanal (7) zwischen der Basis (2) und dem Becherboden (3-2) eine Trennmembran (8) und im Medikamentenmischkanal (7) zwischen der Trennmembran (8) und dem Becherboden (3-2) ein Gummistopfen (6) angeordnet ist;
wobei der Abstand der oberen Fläche des Becherbodens (3-2) zur unteren Fläche der Trennmembran (8) kleiner als die Länge der unteren Nadel (5-3) ist;
wobei der Medikamentenmischer ferner eine Dichtungsmembran (13) umfasst, die an einer Becheröffnung des Medikamentenmischbechers in einer Pressschweißweise verbunden ist, um den Medikamentenmischbecher abzudichten;
wobei die Dichtungsmembran (13) eine leicht zu reißende Membran ist, die nach einer Sterilisation mit feuchter Hitze im Wesentlichen glatt gehalten werden kann;
**dadurch gekennzeichnet, dass**
der Medikamentenmischer eine elastische Klemmbasis (4) umfasst, die einen Klemmring (4-6) aufweist, der an der Unterseite mit einer elastischen Klemmbacke (4-1) versehen ist, wobei die oberen Enden der elastischen Klemmbacke (4-1) gleichmäßig und fest entlang des Klemmrings angeordnet sind und die unteren Enden der elastischen Klemmbacke freie Enden sind, und wobei die elastische Klemmbacke (4-1) von den festen oberen Enden zu den freien unteren Enden in Richtung der Mitte des Medikamentenmischbechers geneigt ist;
wobei die Nadelplatte (5) unterhalb der elastischen Klemmbacke (4-1) im Medikamentenmischbecher angebracht ist;
wobei eine Begrenzungsstruktur an einer Innenwand der Becherwand angeordnet ist, wobei die elastische Klemmbasis (4) in dem Medikamentenmischbecher (3) angebracht ist und durch die Begrenzungsstruktur begrenzt wird;
wobei der Medikamentenmischbecher (3) mit einer bestimmten Menge an Flüssigkeit vorgefüllt wird, bevor er durch die Dichtungsmembran (13) versiegelt wird, um den Luftdruck innerhalb und außerhalb des Medikamentenmischbechers im Wesentlichen auszugleichen; und
wobei die Flüssigkeit schnell in einer Umgebung mit feuchter Hitze sterilisiert werden kann.

2. Medikamentenmischer nach Anspruch 1, wobei die elastische Klemmbasis (4) eine Bodenplatte (4-5), eine Stützsäule (4-4), einen Klemmring (4-6) und eine elastische Klemmbacke (4-1) aufweist, die einstückig ausgebildet sind, wobei die Stützsäule (4-4) an einem Umfang der Bodenplatte (4-5) angeordnet ist, um den Klemmring (4-6) zu stützen und zu befestigen;
wobei ein zentrales Loch auf der Bodenplatte (4-5) angeordnet ist, wobei die untere Nadel durch das zentrale Loch hindurchdringt und dadurch die Quernadel positioniert; und
wobei die Nadelplatte (5-1) zwischen der Bodenplatte (4-5) und der elastischen Klemmbacke (4-1) angeordnet ist.

3. Medikamentenmischer mit einer Verstärkungsstruktur, umfassend den Medikamentenmischer nach einem der Ansprüche 1-2, wobei Verstärkungsrippen (3-3) an der Becherwand (3-1) des Medikamentenmischbechers (3) angeordnet sind, um die Druckfestigkeit der Becherwand zu erhöhen;
wobei die Verstärkungsrippen (3-3) einstückig an einer Innenseite der Becherwand (3-1) in einer Auf- und Abwärtsrichtung und/oder in einer horizontalen Richtung angeordnet sind.

4. Medikamentenmischer nach Anspruch 3, wobei im mittleren Bereich des Gummistopfens (6) ein Durchstichbereich ausgebildet ist, der dünner ist als ein Randbereich;
wobei ein ringförmiger Vorsprung im zentralen Bereich der unteren Oberfläche der Nadelplatte (5-1) angeordnet ist und der Durchstichbereich mit dem durch den ringförmigen Vorsprung begrenzten Bereich überlagert ist oder sich in diesem befindet.

5. Medikamentenmischer nach Anspruch 3, wobei der Medikamentenmischer ferner eine Abdeckplatte (12) aufweist, die in der Becheröffnung angeordnet ist;
wobei ein innerer stufenförmiger Rand entlang der Becherwand an der Becheröffnung angeordnet ist und die Abdeckplatte auf den inneren Rand aufgesetzt ist;
wobei die Oberseite der Abdeckplatte (12) mit der Becheröffnung bündig ist oder etwas tiefer liegt als diese;
wobei die Dichtungsmembran (13) heiß auf die obere Endfläche der Becherwand gepresst wird, wodurch die Becheröffnung abgedichtet wird; und
wobei die Abdeckplatte polygonal ist und einen Loch (12-1) in der Mitte aufweist, wobei die Spitze der oberen Nadel sich in dem Loch befindet und nicht hindurch dringt.

6. Medikamentenmischer nach einem der Ansprüche 3-5, wobei die Dichtungsmembran eine atmungsaktive, leicht zu zerreißende Membran mit einer Luftdurchlässigkeit von 5% bis 35% ist, und
wobei ein Metallfilm auf eine obere Fläche der Dichtungsmembran aufgebracht ist.

7. Medikamentenmischer nach Anspruch 3, wobei das untere Ende des Medikamentenmischkanals mit einem leicht zu öffnenden Griff zum Verschließen des unteren Endes des Medikamentenmischkanals versehen ist.

8. Medikamentenmischer nach Anspruch 3, wobei auf der Oberseite der Trennmembran (8) ein konvexer Ring oder eine konkave Rille angeordnet ist, wobei auf der Unterseite des Gummistopfens (6) entsprechend eine konkave Rille oder ein konvexer Ring angeordnet ist; und wobei die Trennmembran (8) durch den konvexen Ring oder die konkave Rille hindurch mit dem Gummistopfen (6) verschränkt wird.

9. Medikamentenmischer nach Anspruch 3, wobei ein Ausgang des hohlen Kanals der oberen Nadel an der Seitenwand der Spitze der oberen Nadel (5-2) angeordnet ist;
und/oder,
wobei ein Ausgang des hohlen Kanals der unteren Nadel an der Seitenwand der Spitze der unteren Nadel (5-3) angeordnet ist;
wobei die obere Nadel mit einer Schicht aus elastischer Schrumpffolie überzogen ist;
und/oder,
wobei die untere Nadel mit einer Schicht aus elastischer Schrumpffolie beschichtet ist.

10. Doppelte harte Anschluss mit einem Medikamentenmischer, umfassend den Medikamentenmischer nach einem der Ansprüche 1-9, wobei ein Infusionskanal an der Basis des Medikamentenmischers auf der dem Medikamentenmischkanal gegenüberliegenden Seite angeordnet ist; und
wobei Anschlüsse an dem Infusionskanal angeordnet sind.

11. Weicher intravenöser Beutel, umfassend den Medikamentenmischer nach einem der Ansprüche 1-9, oder umfassend den doppelten harten Anschluss nach Anspruch 10,
wobei der Medikamentenmischer oder die doppelten harten Anschluss durch die Basis mit dem weichen Beutel verbunden sind; ferner umfassend einen Infusionsanschluss, der an dem weichen intravenösen Beutel auf derselben Seite wie der Medikamentenmischer oder an dem weichen intravenösen Beutel auf der dem Medikamentenmischer gegenüberliegenden Seite angeordnet ist.

## Revendications

1. Mélangeur de médicament, comprenant une base (2), un passage de mélange de médicament (7) et un godet de mélange de médicament (3) qui sont formés d'un seul tenant, ainsi qu'une aiguille croisée (5), dans lequel le godet de mélange de médicament est constitué d'une paroi de godet (3-1) et d'un fond de godet (3-2) ;
l'aiguille croisée est constituée d'une plaque à aiguille intégrée (5-1), ainsi que d'une aiguille supérieure (5-2) et d'une aiguille inférieure (5-3) avec des passages creux qui communiquent les uns avec les autres ;
dans lequel,
l'extrémité inférieure du passage de mélange de médicament (7) pénètre à travers la base (2) et son extrémité supérieure à travers le fond de godet, une membrane de séparation (8) est agencée dans le passage de mélange de médicament (7) entre la base (2) et le fond de godet (3-2), et un bouchon en caoutchouc (6) est agencé dans le passage de mélange de médicament (7) entre la membrane de séparation (8) et le fond de godet (3-2) ;
la distance entre la surface supérieure du fond de godet (3-2) et la surface inférieure de la membrane de séparation (8) est inférieure à la longueur de l'aiguille inférieure (5-3) ;
le mélangeur de médicament comprend en outre une membrane d'étanchéité (13) qui est reliée par soudage par pression à une embouchure du godet de mélange de médicament, de manière à sceller le godet de mélange de médicament ;
la membrane d'étanchéité (13) est une membrane facilement déchirable qui peut maintenir encore sensiblement lisse après une stérilisation terminale par la chaleur humide ;
**caractérisé en ce que**,
comprenant en outre une base de serrage élastique (4) comportant une bague de serrage (4-6) munie d'une mâchoire de serrage élastique (4-1) au niveau du côté inférieur, les extrémités supérieures de la mâchoire de serrage élastique (4-1) sont agencées de manière uniforme et fixe le long de la bague de serrage, et les extrémités inférieures de la mâchoire de serrage élastique sont des extrémités libres, et la mâchoire de serrage élastique (4-1) s'incline vers le centre du godet de mélange de médicament depuis ses extrémités supérieures fixes jusqu'à ses extrémités inférieures libres ;
la plaque à aiguille (5) est montée sous la mâchoire de serrage élastique (4-1) dans le godet de mélange de médicament ;
une structure de limitation est agencée sur un côté intérieur de la paroi de godet, et la base de serrage élastique (4) est montée à l'intérieur du godet de mélange de médicament (3) et est limitée par la structure de limitation ;
le godet de mélange de médicament (3) est prérempli d'une certaine quantité de liquide avant d'être scellé par la membrane d'étanchéité (13), pour équilibrer sensiblement la pression d'air à l'intérieur et à l'extérieur du godet de mélange de médicament ;
le liquide peut être rapidement vaporisé dans un environnement de stérilisation par la chaleur humide.

2. Mélangeur de médicament selon la revendication 1, **caractérisé en ce que**,
la base de serrage élastique (4) comporte une plaque de fond (4-5), une colonne de support (4-4), une bague de serrage (4-6) et une mâchoire de serrage élastique (4-1), qui sont formées d'un seul tenant, dans laquelle la colonne de support (4-4) est agencée sur une périphérie de la plaque de fond (4-5), de manière à supporter et à fixer la bague de serrage (4-6) ;
un trou central est agencé sur la plaque de fond (4-5), l'aiguille inférieure pénètre à travers le trou central, ce qui permet de positionner l'aiguille croisée ;
la plaque à aiguille (5-1) est située entre la plaque de fond (4-5) et le mâchoire de serrage élastique (4-1).

3. Mélangeur de médicament ayant une structure de renfort, comprenant le mélangeur de médicament selon l'une quelconque des revendications 1 à 2 ;
des nervures de renfort (3-3) sont agencées sur la paroi (3-1) du godet de mélange de médicament (3), de manière à améliorer la résistance à la compression de la paroi de godet ;
les nervures de renfort (3-3) sont agencées intégralement sur un côté intérieur de la paroi de godet (3-1) dans une direction ascendante et descendante et/ou une direction horizontale.

4. Mélangeur de médicament selon la revendication 3, **caractérisé en ce que**,
une zone à perforation est formée dans la zone centrale du bouchon en caoutchouc (6), et est plus mince qu'une zone marginale ;
une saillie annulaire est agencée dans la zone centrale de la surface inférieure de la plaque à aiguille (5-1), et la zone à perforation est superposée à ou située dans la zone limitée par la saillie annulaire.

5. Mélangeur de médicament selon la revendication 3, **caractérisé en ce que**,
le mélangeur de médicament comprend en outre une couvre-plaque (12) ; la couvre-plaque est située au niveau de l'embouchure du godet ;
un bord intérieur étagé est agencé le long de la paroi de godet au niveau de l'embouchure du godet, et la couvre-plaque est posée sur le bord intérieur ;
la surface supérieure de la couvre-plaque (12) est au même niveau ou légèrement plus basse que l'embouchure du godet ;
la membrane d'étanchéité (13) est pressée à chaud sur la surface d'extrémité supérieure de la paroi de godet, scellant ainsi l'embouchure du godet ;
la couvre-plaque est polygonale, avec un trou (12-1) au centre, et la pointe de l'aiguille supérieure est située dans le trou et ne le traverse pas.

6. Mélangeur de médicament selon l'une quelconque des revendications 3 à 5, **caractérisé en ce que**,
la membrane d'étanchéité est une membrane respirante et facile à déchirer, avec une perméabilité à l'air de 5% à 35% ;
un film métallique est plaqué sur une surface supérieure de la membrane d'étanchéité.

7. Mélangeur de médicament selon la revendication 3, **caractérisé en ce que**, l'extrémité inférieure du passage de mélange de médicament est munie d'une poignée facile à briser pour sceller l'extrémité inférieure du passage de mélange de médicament.

8. Mélangeur de médicament selon la revendication 3, **caractérisé en ce que**,
un bague convexe ou une rainure concave est agencée sur la surface supérieure de la membrane de séparation (8), et une rainure concave ou un bague convexe est agencée de manière correspondante sur la surface inférieure du bouchon en caoutchouc (6) ;
la membrane de séparation (8) est posée sur le bouchon en caoutchouc (6) au moyen de la bague convexe ou de la rainure concave.

9. Mélangeur de médicament selon la revendication 3, **caractérisé en ce que**,
une sortie du passage creux de l'aiguille supérieure est agencée sur la paroi latérale de la pointe de l'aiguille supérieure (5-2) ;
et/ou,
une sortie du passage creux de l'aiguille inférieure est agencée sur la paroi latérale de la pointe de l'aiguille inférieure (5-3) ;
l'aiguille supérieure est revêtue d'une couche de film rétractable élastique ;
et/ou,
l'aiguille inférieure est revêtue d'une couche de film rétractable élastique.

10. Doubles orifices durs avec un mélangeur de médicament, comprenant le mélangeur de médicament selon l'une quelconque des revendications 1 à 9, **caractérisé en ce que**, un passage de perfusion est agencé sur la base du mélangeur de médicament, sur le côté opposé au passage de mélange de médicament ; et
Les orifices sont agencés sur le passage de perfusion.

11. Poche de perfusion souple, comprenant le mélangeur de médicament selon l'une quelconque des revendications 1 à 9 ;
ou comprenant les doubles orifices durs selon la revendication 10 ;
le mélangeur de médicament ou les doubles orifices durs sont reliés à la poche souple par la base ;
comprenant en outre un orifice de perfusion qui est agencé sur la poche de perfusion souple du même côté que le mélangeur de médicament, ou agencé sur la poche de perfusion souple du côté opposé au mélangeur de médicament.
